Europäisches Patentamt

European Patent Office

Office européen des brevets

(11) Publication number: **0 512 120 A1**

## EUROPEAN PATENT APPLICATION
## published in accordance with Art. 158(3) EPC

(21) Application number: **92902480.0**

(22) Date of filing: **22.11.91**

(86) International application number:
**PCT/JP91/01606**

(87) International publication number:
**WO 92/09692 (11.06.92 92/13)**

(51) Int. Cl.⁵: **C12N 15/31**, C12N 1/21,
//(C12N15/31,C12R1:38),
(C12N1/21,C12R1:38)

(30) Priority: **22.11.90 JP 316044/90**
**18.11.91 JP 328393/91**

(43) Date of publication of application:
**11.11.92 Bulletin 92/46**

(84) Designated Contracting States:
**DE FR GB IT**

(71) Applicant: **Japan Tobacco Inc.**
**4-12-62 Higashishinagawa**
**Shinagawa-ku, Tokyo 140(JP)**

(72) Inventor: **NEGISHI, Hideaki, Yokohama Center**
**of Appl. Plant**
**-Research Lab., Japan Tobacco Inc. 6-2,**
**Umegaoka,**
**Midori-ku, Yokohama-shi, Kanagawa 227(JP)**
Inventor: **TANAKA, Hiroshi, Yokohama Center**
**of Appl. Plant**
**Res. Laboratory, Japan Tobacco Inc. 6-2,**
**Umegaoka,**
**Midori-ku, Yokohama-shi, Kanagawa227(JP)**
Inventor: **YAMADA, Tetsuji, Okayama**
**University,**
**College of Agriculture 1-1-1, Tsushima-naka**
**Okayama-shi Okayama700(JP)**

(74) Representative: **Reinhard, Skuhra, Weise**
**Postfach 44 01 51 Friedrichstrasse 31**
**W-8000 München 44(DE)**

(54) **PLASMID FOR USE IN REMOVING PATHOGENICITY AND NONPATHOGENIC TRANSFORMANT.**

(57) A plasmid which can provide nonpathogenic bacteria of the genus *Pseudomonas* readily and stably. It is derived from M4S (FERM BP-700) which is a nonpathogenic mutant of *Pseudomonas solanacearum* and it can render pathogenic bacteria of the genus *Pseudomonas* nonpathogenic and nontoxic.

EP 0 512 120 A1

Field of the Invention

This invention relates to a plasmid for use in removing pathogenicity and, more particularly, a plasmid derived from M4S strain which is a nonpathogenic mutant strain of Pseudomonas solanacearum, a plant pathogenic bacterium, which can remove pathogenicity of pathogenic bacteria belonging to the genus Pseudomonas to detoxify them.

Furthermore, this invention relates to a nonpathogenic transformant which is obtained by introducing the said plasmid into a bacteria belonging to the genus Pseudomonas for use in removing pathogenicity.

Prior Art

Bacteria belonging to Pseudomonas are the bacteria which cause bacterial grain rot of rice, bacterial wilt of solanaceous plant, bacterial wilt of tobacco or other bacterial diseases and damages caused by these diseases are one of the most serious agricultural problems. Biological control of these diseases by using nonpathogenic strains of the same spieces has been tried.

On the other hand, certain bacteria belonging to the genus Pseudomonas show competitive effect against soil-borne plant pathogens and they are tried to use as biological control agents and are used practically in certain applications.

These bacteria for biological control, however, are spontaneous mutants or are derived from the selection from the soil.

Methods depending on such mutation or selection consume much labor and one can not always obtain bacteria provided with all the required properties.

Therefore, the gene manipulation may be proposed as a more rational selection method, but the process which can remove pathogenicity of pathogens among bacteria belonging to the genus Pseudomonas is not found except rather random method of transposon and others.

Moreover, certain bacteria belonging to the genus Pseudomonas are notorious for causing human infectious diseases and others and it is believed to be important to remove their pathogenicity from the medical point of view.

Therefore, the object of the present invention is to provide nonpathogenic bacteria belonging to the genus Pseudomonas which can be supplied easily and regularly.

Disclosure of the Invention

Inventors of the present invention have made different examinations of bacteria belonging to the genus Pseudomonas which are plant pathogenic bacteria.

As the result, the inventors have succeeded in isolating a plasmid which does not exist in the pathogenic wild strain from the nonpathogenic mutant M4S strain of Pseudomonas solanacearum and, moreover, in removing its pathogenicity by introducing this plasmid accompanied with drug resistance into bacteria belonging to the genus Pseudomonas.

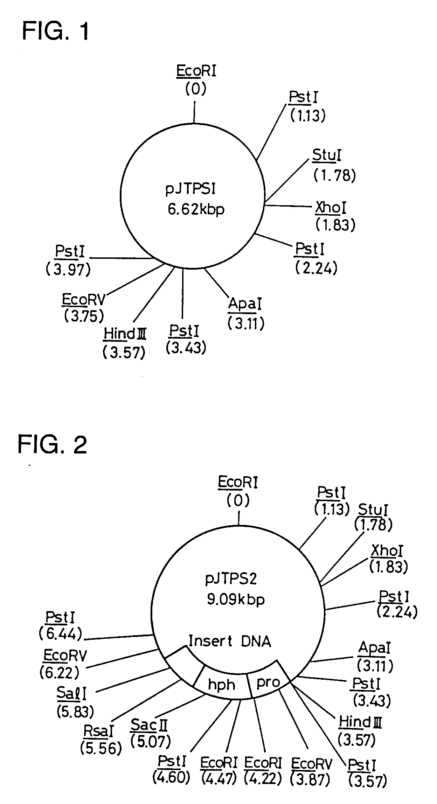

The plasmid of the present invention is a plasmid derived from M4S, nonpathogenic mutant of Pseudomonas solanacearum, characterized by the fact that it can remove pathogenicity of pathogenic bacteria belonging to the genus Pseudomonas and detoxify them and preferably, the same has the molecular weight of approximately 6.62 kbp and has the restriction endonuclease map as shown in Fig.1.

Pseudomonas solanacearum M4S had been deposited as FERM P-7370 on December 14, 1983 with the Fermentation Research Institute, Agency of Industrial Science and Technology, the Ministry of International Trade and Industry located at 1-3, Higashi 1-chome, Yatabe-machi, Tsukuba-gun, Ibaraki-ken, Japan (Postal Code Number 305), and was transferred to the International deposit in the same on January 24, 1985 and given as modified accession number FERM BP-700.

Moreover, the plasmid wherein drug resistant gene and promoter gene of the same are supplementary integrated into the said plasmid is also included in the scope of the invention and it has, preferably, the molecular weight of approximately 9.09 kbp and has the restriction endonuclease map as shown in Fig.2.

The nonpathogenic transformant of the invention is a bacterium belonging to the genus Pseudomonas, having one of the said plasmids in its cell.

The said plasmids can be isolated from this bacterium by means of a conventional process.

For example, at first, the shake culture of the bacteria is performed in a liquid medium of about pH7 to 8 at 28 to 30°C during about 10 to 24 hours using nutrient such as Casamino acid (trade name of Difco Inc., a hydrolysate of casein by acid), peptone, glucose and others.

Then, the cultivated bacteria is lysed by an alkaline surfactant and polyethylene glycol is added to precipitate and agglutinate the cellular content.

The protein is removed from this precipitate by a conventional method, then, after the collection of nucleic acid by alcohol precipitation, the same is purified by high performance liquid chromatography using hydroxylapatite column to obtain the desired plasmid.

This plasmid is a circular double-stranded DNA having the molecular weight of approximately 6.62 kbp and the restriction endonuclease map shown in Fig.1 (hereinafter, this plasmid may be referred to as pJTPS1).

Then, gene that plays as a marker is integrated in the plasmid obtained above.

In this step, drug resistant genes are ordinarily used as markers.

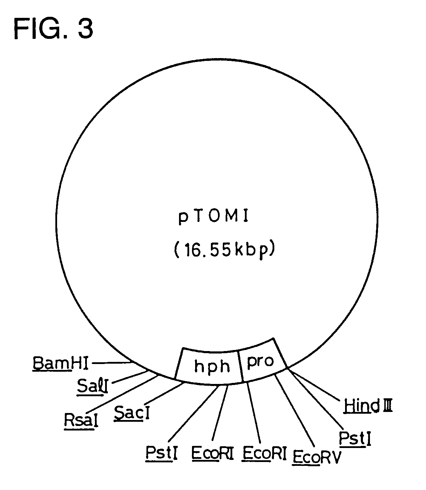

For instance, when it is desired to integrate hygromycin resistant gene as a marker, the obtained plasmid is cleaved by restriction endonuclease HindIII and hygromycin resistant gene containing promoter gene derived from the plasmid pTOM1 [by Nishino, Yamada, Shiraishi and Oku (Laboratory of Plant Pathology Faculty of Agriculture, Okayama University)] shown in Fig.3 is linked to the cleavage site.

In Fig.3, hph indicates hygromycin gene and pro indicates promoter gene respectively.

This higromycin resistant gene is a fragment of approximately 2.45 kbp which is cleaved by the restriction endonucleases HindIII and BamHI.

The plasmid integrated with marker is introduced into bacteria belonging to the genus Pseudomonas by a conventional method. In this process, the bacteria is transformed and nonpathogenic bacteria can be obtained.

The plasmid extracted from this transformant by the same method as mentioned above is a circular double-stranded DNA having the molecular weight of approximately 9.09 kbp and the restriction endonuclease map shown in Fig.2 (hereinafter, this plasmid may be referred to as pJTPS2).

Brief Description of Drawings

Fig.1 shows the restriction endonuclease fragmentation map of the plasmid pJTPS1 according to the present invention.

Fig.2 shows the restriction endonuclease fragmentation map of the plasmid pJTPS2 according to the present invention.

Fig.3 is the restriction endonuclease fragmentation map showing the location of hygromycin resistant gene in the plasmid pTOM1.

Description of the Preferred Embodiments

The present invention shall be described more in detail referring to the following embodiments, which should not be understood to limit the invention.

(1) Culture of bacteria

Pseudomonas solanacearum M4S is inoculated in 1 ml of CPG liquid medium (0.1% Casamino acid, 1% peptone, 1% glucose) and is submitted to the shake culture at 30°C for about 18 hours.

Thereafter it is successively inoculated in 250 ml of CPG liquid medium and is further submitted to the shake culture for about 18 hours.

(2) Preparation of plasmid

The culture broth obtained in the said step (1) is centrifuged at 6500 rpm for 20 minutes to collect the bacteria.

The bacteria is washed with 15 mM-NaCl, suspended again in 20 ml of 15 mM-NaCl, then 140 ml of lysate (3% SDS, 50 mM of tris hydrochloric acid, 30 mM EDTA, 5 mM NaCl, pH 12.45) is added before leave it for 10 minutes at 50 °C.

Then, the pH is readjusted to 8 to 9 with 2 M of tris hydrochloric acid (pH 5.5) agitating gently; thereafter, 50 ml of 5M-NaCl is added and mixed gently.

Then the solution is left on the ice more than one hour before being centrifuged at 15000 rpm, 4 °C for 15 minutes.

After centrifugation, the supernatant is collected, filtered with kimwipe, mixed with PEG solution [60% polyethylene glycol 6000, 10 mM tris hydrochloric acid (pH8.0)] of the volume corresponding to one fifth of the same and then left on the ice for more than one hour.

The same is centrifuged at 15000 rpm, 4 °C for 30 minutes and the supernatant is poured out to collect the precipitate.

The precipitate thus obtained is solved with a small amount of TEN buffer solution [50 mM tris hydrochloric acid (pH 8.0), 30 mM EDTA, 5 mM NaCl], removed of protein through phenol-chloroform treatment, desalted through ethanol precipitation and then rinsed out.

In this step, the phenol-chloroform treatment is proceeded as follows.

First, equivalent volume of TE buffer solution saturated phenol-chloroform-isoamyl alcohol (25:24:1) is added, shaken and centrifuged to collect aqueous phase.

The same procedure is repeated several times, then equivalent volume of chloroform-isoamyl alcohol (24:1) is further added, shaken to collect aqueous phase.

Ethanol precipitation is carried out as follows.

First, 0.1 times volume of 3 M-sodium acetate and 2.5 times volume of ethanol are added, agitated and left at -70 °C for 30 minutes.

Then, the same is centrifuged at 15000 rpm, 4 °C for 30 minutes to precipitate DNA and remove the supernatant.

Thereafter, the same is rinsed out with 70% ethanol and dried.

DNA thus obtained is dissolved in 2 ml of sterilized distilled water and the plasmid is purified using high performance liquid chromatography.

Parameters concerning the high performance liquid chromatography are as follows.

Apparatus          : Hitachi 655.15
Detection          : UV 260 nm
Column             : Hydroxylapatite charged column (Mitsui Toatsu Chemicals, Inc. HCA-column A-8010G)
Isolation medium   : potassium phosphate buffer solution
                     0 to 20 minutes 100 mM
                     20 to 30 minutes 200 mM
                     30 to 50 minutes 300 mM
Flow rate          : 1.0 ml/minute
Temperature        : Room temperature
Sample amount      : 1 ml

Part of plasmid eluted in a fraction of 200 mM is sampled and dialyzed one night at 4 °C against TE buffer solution to remove phosphoric acid. The same is desalted through ethanol precipitation, concentrated before being dissolved in TE buffer solution and stored at -20 °C.

Plasmid can also be purified through isopycnic density gradient centrifugation using cesium chloride.

(3) Cleavage with restriction endonuclease

The molecular weight of plasmid pJTPS1 obtained in the step (2) hereabove is calculated and the plasmid is cleaved using different restriction endonucleases to make a restriction endonuclease map.

The cleavage is carried out using restriction endonucleases produced by Takara Shuzo Co. Ltd. and Nippon Gene Inc. in accordance with the cleavage conditions specified by the manufacturers.

Cleaved samples are submitted to the agarose gel electrophoresis.

First, cleaved samples are placed respectively on 0.7%, 1.2% and 2% agarose gel containing 0.5 $\mu$g/ml of ethidium bromide, subjected to electrophoresis in TBE buffer solution containing 0.5 $\mu$g/ml of ethidium bromide at a constant voltage of 50 V for 2 to 4 hours before separating fragments.

Fragments cleaved by restriction endonucleases are detected by ultraviolet irradiation (300 nm) followed by photographing them.

The molecular weight of the plasmid pJTPS1 calculated from fragments by restriction endonuclease is approximately 6.62 kbp.

As molecular weight of the fragment cleaved determinating marker, fragments obtained by digesting lambda phage's DNA with the restriction endonuclease HindIII or StyI are used.

The sensitivity of the plasmid pJTPS1 to each of different restriction endonucleases determined in this manner are shown in Table 1.

The restriction endonuclease fragmentation map of this plasmid is as shown in Fig.1.

In Fig.1, the restriction sites are shown in kbp (kilobase pairs) taking EcoRI restriction site as the origin of coordinates.

Table 1

| Restriction endonuclease | Number of cleavage | Restriction sites (kpb) |
|---|---|---|
| ApaI | 1 | 3.11 |
| EcoRI | 1 | 0/6.62 |
| EcoRV | 1 | 3.75 |
| HindIII | 1 | 3.57 |
| PstI | 4 | 1.13/2.24 3.43/3.97 |
| StuI | 1 | 1.78 |
| XhoI | 1 | 1.83 |
| AccI | 2 | Not measured |
| BglII | 2 | Not measured |
| SalI | 2 | Not measured |

(4) Construction of recombinant DNA

a) Preparation of insert DNA (promoter gene and hygromycin resistant gene)

Plasmid pTOM1 is digested with restriction endonucleases HindIII and BamHI and subjected to low-melting temperature agarose gel electrophoresis.

First, cleaved samples are placed on 0.7% low temperature melting agarose gel containing 0.5 $\mu$g/ml of ethidium bromide.

Second, the same is subjected to electrophoresis in TBE buffer solution containing 0.5 $\mu$g/ml of ethidium bromide at a constant voltage of 50 V for 2 to 4 hours before separating fragments.

The position of the separated DNA fragments is detected under 360 nm ultraviolet irradiation and gel portion containing the target fragment of approximately 2.47 kbp is cut out using a razor blade, maintained at 65 °C and melted completely.

Two times of TE buffer is added thereto and the same is subjected to phenol-chroloform treatment, and further to ehtyl ether treatment.

Thereafter, ethanol precipitation is carried out before rinsing and drying the same.

Here, the ehtyl ether treatment is carried out in accordance with the following steps.

First, equivalent volume of ethyl ether is added, shaken and centrifuged to collect aqueous layer.

This step is repeated three times, and the remaining ehtyl ether is removed using nitrogen gas.

b) Cleavage of vector DNA (pJTPS1)

The plasmid obtained in the step (2) hereabove is cleaved with restriction endonuclease HindIII and one tenth volume of 10% SDS and 0.5 M EDTA are added thereto before heating at 75 °C for 15 minutes.

The same is subjected to phenol-chroloform treatment, and further to ethyl ether treatment, then ethanol precipitation is carried out to rinse it before drying.

c) Ligation of insert DNA and vector DNA

The DNA obtained in the step a) and b) hereabove are ligated by T4 DNA ligase and one tenth volume of 10 % SDS and 0.5 M EDTA are added thereto before heating at 75 °C for 15 minutes.

The same is subjected to phenol-chroloform treatment, and futher to ethyl ether treatment, then ethanol precipitation is carried out to rinse it before drying.

Then the two ends of DNA are made blunt ends by using E. coli DNA polymerase I (large fragment) and one tenth volume of 10% SDS and 0.5 M EDTA are added and heated at 75 °C for 15 minutes, before carrying out phenol-chloroform treatment and ethyl ether treatment. Thereafter, it is subjected to ethanol precipitation to rinse and dry it out.

After that, the two ends of DNA are ligated by T4 DNA ligase to obtain circular double-stranded DNA (plasmid pJTPS2).

Here, T4 DNA ligase and E. coli DNA polymerase I (large fragment) made by Takara Shuzo Co. Ltd. are used in accordance with the reaction parameters specified by the manufacturer.

(5) Transformation of Pseudomonas solanacearum

Using DNA obtained in (4) above, the transformation of pathogenic wild strain U-7 belonging to Pseudomonas solanacearum using the following method described by Boucher et al. [Journal of General Microbiology, vol. 131, 2449-2457 (1985)].

First, 50 $\mu$l of U-7 bacteria cultivated up to 5 x $10^8$ at 30 °C in MM medium [M63 medium of 1/4 concentration (Maniatis et al., 1982, Molecular Cloning, A Laboratory Manual)] added with glucose (0.2%) and glycerol (4 ml/l) are mixed with DNA 7.65 $\mu$g obtained in (4) hereabove and transferred to MM agar medium added with glycerol (4 ml/l).

Having been cultivated at 30 °C for 48 to 60 hours, cells are suspended again into 20 ml of sterilized water, 100 $\mu$l thereof is spread on CPG agar medium supplemented with hygromycin (30 ppm) and 2,3,5 triphenyl tetrazolium chloride (0.05 %) and cultured at 30 °C from 24 to 72 hours.

The number of appeared colonies resistant to hygromycin is counted in order to determinate the transformation efficiency.

Under the parameters mentioned hereinabove, the transformation efficiency is approximately 1.2 x $10^4$ colony per DNA 1 $\mu$g.

The transformant obtained hereabove had been deposited as FERM P-11746 on September 28, 1990 with the Fermentation Research Institute, Agency of Industrial Science and Technology, the Ministry of International Trade and Industry located at 1-3, Higashi 1-chome, Yatabe-machi, Tsukuba-gun, Ibaraki-ken, Japan (Postal Code Number 305), and was transferred to the International deposit in the same on November 11, 1991 and given as modified accession number FERM BP-3650.

(6) Extraction, purification and verification of recombinant DNA

From hygromycin resistant bacteria appeared in the steps (5) hereabove, plasmid is extracted and purified in the same manner as in (1) and (2) mentioned before.

On thus obtained plasmid, recombinant DNA is confirmed by Southern blotting method which uses hygromycin resistant gene as a probe.

Here, the probe is created using Random Primer Labeling Kit manufactured by Takara Shuzo Co. Ltd. in accordance with the conditions specified by the manufacturer.

Further, in order to verify the orientation of the inserted DNA, the analysis is carried out by the same method as in (3) hereabove using restriction endonucleases HindIII and EcoRV.

The restriction endonuclease map of plasmid pJTPS2 plotted in this way is as shown in Fig.2.

In Fig.2, cleavage positions are shown in kbp taking EcoRI cleavage position as the origin of coordinates.

As for cleavage positions of the restriction endonucleases RsaI, SacII and SalI, only insert DNA portions are shown.

(7) Properties of transformant

a) Colony morphology

Transformant obtained in (5) hereabove, M4S bacteria and U-7 bacteria are spread over CPG agar medium added with 2,3,5 triphenyl tetrazolium chloride (0.05%), cultured 24 to 72 hours at 30 °C in order to compare the colony shape.

U-7 bacteria shows the fluidal colony shape which is typical for pathogenic bacteria and the transformants, on the other hand, show non-fluidal colony shape which is typical for non pathogenic bacteria as all M4S bacteria.

b) Pathogenicity against Tomato -1

Transformants obtained in (5) hereabove, M4S bacteria and U-7 bacteria are inoculated to wound made on tomato seedling one week (seven days) after the sowing.

Pathogenicity of each strain against tomato one week after the sowing is shown in Table 2. In Table 2, the asterisk (*) indicates "transformant".

As shown in Table 2, all tomatoes (100%) inoculated with U-7 bacteria were infected with the ddisease and wilted after one week; on the other hand, transformants and M4S bacteria did not show any symptom of disease.

Table 2

| Strain | Disease incidence (%) |
|---|---|
| U-7 | 100 |
| M4S | 0 |
| U-7: pJTPS2 -1* | 0 |
| U-7: pJTPS2 -2* | 0 |
| U-7: pJTPS2 -3* | 0 |
| U-7: pJTPS2 -4* | 0 |

c) Pathogenicity against Tomato -2

Transformants obtained in (5) hereabove, M4S bacteria and U-7 bacteria are inoculated to wound made on tomato seedling 4 weeks after the sowing.

Pathogenicity of each strain against tomato 4 weeks after the sowing is shown in Table 3. In Table 3, the asterisk (*) indicates "transformant".

As shown in Table 3, all tomatoes (100%) inoculated with U-7 bacteria were infected with the disease and wilted after 1 week; on the other hand, transformants and M4S did not show any symptom of disease.

Table 3

| Strain | Disease incidence (%) |
|---|---|
| U-7 | 100 |
| M4S | 0 |
| U-7: pJTPS2 -1* | 0 |
| U-7: pJTPS2 -2* | 0 |
| U-7: pJTPS2 -3* | 0 |
| U-7: pJTPS2 -4* | 0 |

d) Pathogenicity against Tobacco

Transformants obtained in (5) hereabove, M4S bacteria and U-7 bacteria are inoculated by infiltrating in the leaf tissue of tobacco 10 weeks after the sowing.

As the result, tobaccos inoculated with U-7 bacteria were infected with the disease and wilted; on the other hand, transformants and M4S bacteria have shown no symptom of disease.

As it is evident from the results of beforementioned a) to d), the transformants obtained through plasmid pJTPS2 are completely removed of pathogenicity.

Industrial applicability

As described above, plasmid pJTPS1 of the present invention can remove pathogenicity from the pathogenic bacteria belonging to the genus Pseudomonas and it can be used advantageously as vector to create bacteria for the biological control.

Further, as plasmid pJTPS2 is integrated into pJTPS1 such that it can express hygromycin resistant gene, it can be used advantageously as a vector to create easily bacteria for the biological control.

Therefore, the transformants of bacteria belonging to the genus Pseudomonas of the invention in which one of the said plasmids is introduced are nonpathogenic.

**Claims**

1. A plasmid derived from M4S (FERM BP-700) which is a nonpathogenic mutant of Pseudomonas solanacearum, wherein the plasmid can remove and detoxify the pathogenicity of pathogenic bacteria belonging to the genus Pseudomonas.

2. The plasmid according to claim 1 having cleavage positions at 1.13 kbp by PstI, at 1.78 kbp by StuI, at 1.83 kbp by XhoI, at 2.24 kbp by PstI, at 3.11 kbp by ApaI, at 3.43 kbp by PstI, at 3.57 kbp by HindIII, at 3.75 kbp by EcoRV and at 3.97 kbp by PstI starting from the cleavage position by EcoRI and having the molecular weight of approximately 6.62 kbp.

3. The plasmid according to claim 1 wherein drug resistant genes and further promoter genes of the said genes are integrated.

4. The plasmid according to claim 3 having cleavage positions at 1.13 kbp by PstI, at 1.78 kbp by StuI, at 1.83 kbp by XhoI, at 2.24 kbp by PstI, at 3.11 kbp by ApaI, at 3.43 kbp by PstI, at 3.57 kbp by HindIII, at 3.57 kbp by PstI, at 3.87 kbp by EcoRV, at 4.22 kbp by EcoRI, at 4.47 kbp by EcoRI, at 4.60 kbp by PstI, at 5.07 kbp by SacII, at 5.56 kbp by RsaI, at 5.83 kbp by SalI, at 6.22 kbp by EcoRV and at 6.44 kbp by PstI starting from the cleavage position by EcoRI and having the molecular weight of approximately 9.09 kbp.

5. A nonpathogenic transformant of bacteria belonging to the genus Pseudomonas, wherein the said nonpathogenic transformant includes the said plasmid of claim 1 in its cell.

6. A nonpathogenic transformant of bacteria belonging to the genus Pseudomonas, wherein the said nonpathogenic transformant includes the said plasmid of claim 3 in its cell.

## FIG. 1

pJTPSI
6.62kbp

EcoRI (0)
PstI (1.13)
StuI (1.78)
XhoI (1.83)
PstI (2.24)
ApaI (3.11)
PstI (3.43)
HindIII (3.57)
EcoRV (3.75)
PstI (3.97)

## FIG. 2

pJTPS2
9.09kbp

Insert DNA
hph
pro

EcoRI (0)
PstI (1.13)
StuI (1.78)
XhoI (1.83)
PstI (2.24)
ApaI (3.11)
PstI (3.43)
HindIII (3.57)
PstI (3.57)
EcoRV (3.87)
EcoRI (4.22)
EcoRI (4.47)
PstI (4.60)
SacII (5.07)
RsaI (5.56)
SaII (5.83)
EcoRV (6.22)
PstI (6.44)

# FIG. 3

# INTERNATIONAL SEARCH REPORT

International Application No. PCT/JP91/01606

**I. CLASSIFICATION OF SUBJECT MATTER (if several classification symbols apply, indicate all)** 6

According to International Patent Classification (IPC) or to both National Classification and IPC

Int. Cl$^5$  C12N15/31, 1/21// (C12N15/31, C12R1:38),
(C12N1/21, C12R1:38)

**II. FIELDS SEARCHED**

Minimum Documentation Searched 7

| Classification System | Classification Symbols |
|---|---|
| IPC | C12N15/00–15/90, C12N1/21 |

Documentation Searched other than Minimum Documentation
to the Extent that such Documents are Included in the Fields Searched 8

Biosis Database, Chemical Abstract Database (CA, REG)

**III. DOCUMENTS CONSIDERED TO BE RELEVANT** 9

| Category* | Citation of Document, 11 with indication, where appropriate, of the relevant passages 12 | Relevant to Claim No. 13 |
|---|---|---|
| X/Y | J. Bacteriol., Vol. 170, No. 2, 1988, Sequeira L. et al. "Molecular cloning of genes that specify virulence in Pseudomonas-solanacearum" p. 617-622 | 1-6 |
| X/Y | J. Bacteriol., Vol. 169, No. 12, 1987, Zischek C. et al. "Psendomonas-Solanacearum genes controlling both pathogenicity on tomato and hypersensitivity on tobacco are clustered" p.5626-5632 | 1-6 |
| X/Y | Mol. Gen. Genet., Vol. 205, No. 2, 1986, Zischek C. et al. "Virulence genes are carried by a megaplasmid of the plant pathogen Pseudomonas-solanacearum" p. 270-275 | 1-6 |
| X/Y | J. Bacteriol., Vol. 172, No. 9, 1990, Carney B. F. et al. "A cloned avirulence gene from Pseudomonas-Solanacearum determines | 1-6 |

Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| February 10, 1992 (10. 02. 92) | February 25, 1992 (25. 02. 92) |

| International Searching Authority | Signature of Authorized Officer |
|---|---|
| Japanese-Patent Office | |

FURTHER INFORMATION CONTINUED FROM THE SECOND SHEET

| | |
|---|---|
| | incompatibility on Nicotiana tabacum at the host species level" p: 4836-4843 |
| X/Y | JP, A, 63-22178 (Daikin Industries, Ltd.),      5-6 January 29, 1988 (29. 01. 1988), (Family: none) |

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers      because they relate to subject matter not required to be searched by this Authority, namely:

2.☐ Claim numbers      because they relate to parts of the international application that do not comply with the prescribed requirements to such an extent that no meaningful international search can be carried out, specifically:

3.☐ Claim numbers      because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first-mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)